# EUROPEAN PATENT APPLICATION

(11) **EP 4 844 051 A1**
(43) Date of publication of application: **25.11.2026**
(21) Application number: 25761796.9
(22) Date of filing: 09.01.2025
(51) Int. Cl.: C12M 1/04, C12M 1/36

(54) **CULTURE APPARATUS**

(30) Priority: 29.02.2024 JP 2024030603
(71) Applicant: FUJIFILM Corporation, Tokyo 106-8620 (JP)
(72) Inventor: YOSHIDA, Shunichi, Ashigarakami-gun, Kanagawa 258-8577 (JP); SHIMODA, Yasuhisa, Ashigarakami-gun, Kanagawa 258-8577 (JP); TAKASE, Masanori, Ashigarakami-gun, Kanagawa 258-8577 (JP)
(74) Representative: Hoffmann Eitle
(86) International application number: PCT/JP2025/000542
(87) International publication number: WO 2025/182311

(57) **Abstract**

A culture apparatus for culturing a microorganism or a cell using a combustible raw material gas includes a culture tank, a circulation path that takes out a culture solution from an outlet port provided in the culture tank and returns the culture solution to the culture tank through a return port, a pump that circulates the culture solution through the circulation path and generates a vertically downward flow of the culture solution in the circulation path, and a bubble generating device that generates bubbles having a diameter of 100 µm or more and supplies the raw material gas to the circulation path as bubbles. The outlet port is disposed vertically above the return port, and the bubble generating device is disposed upstream of a midpoint of a length from the outlet port to the return port.

## Description

### BACKGROUND OF THE INVENTION

### 1. Field of the Invention

The disclosed technology relates to a culture apparatus.

### 2. Description of the Related Art

In order to reduce greenhouse gas emissions, research on reducing reliance on petroleum-based raw materials is actively conducted worldwide. As part of such research, in the field of product manufacturing such as chemical products and bioproducts, a technology for using a carbon source other than petroleum as a raw material has been developed. For example, a microorganism that uses a carbon source other than petroleum as a raw material for proliferation or production of an organic substance is known. An example of such a microorganism is hydrogen-oxidizing bacteria. The hydrogen-oxidizing bacteria proliferate by taking in a gas containing carbon dioxide, hydrogen, and oxygen as a raw material, and further efficiently produce an organic substance such as alcohol and an amino acid by modifying a gene of the hydrogen-oxidizing bacteria. The hydrogen-oxidizing bacteria are a general term for chemosynthetic bacteria that use energy generated by oxidation of hydrogen to convert carbon contained in carbon dioxide into an organic substance and incorporate the organic substance into the bacteria. The hydrogen-oxidizing bacteria are also attracting attention in that carbon dioxide, which is emitted as a greenhouse gas, can be effectively used, because carbon dioxide serves as a raw material for proliferation or production of an organic substance.

JP1976-038480A (JP-S51-038480A) and US2019/0316072A disclose a culture apparatus that cultures hydrogen-oxidizing bacteria. The culture apparatus includes a culture tank that contains a culture solution. A raw material gas containing carbon dioxide, hydrogen, and oxygen is supplied into the culture tank, and a part of the carbon dioxide, the hydrogen, and the oxygen contained in the raw material gas is dissolved in the culture solution and is consumed for proliferation of the hydrogen-oxidizing bacteria or production of an organic substance. The remaining raw material gas is released into a space present above a liquid surface of the culture solution in the culture tank and is exhausted to the outside of the culture tank. Since a mixed gas containing hydrogen, which is a combustible gas, and oxygen may be dangerous in terms of explosion depending on a compositional ratio, JP1976-038480A (JP-S51-038480A) and US2019/0316072A disclose a technology for suppressing explosion.

In JP1976-038480A (JP-S51-038480A), the dissolved oxygen concentration in the culture solution is measured after the supply of oxygen contained in the raw material gas is independently controlled, and the amount of oxygen supplied into the culture tank is limited based on the measured dissolved oxygen concentration. In US2019/0316072A, safety is ensured by controlling components of the raw material gas.

In addition, in the culture apparatuses disclosed in JP2016-002040A and JP2018-117578A, a bubble generating device that generates microbubbles or nanobubbles, which are fine bubbles having a diameter of less than 100 µm, is used, and the raw material gas is supplied to the culture solution as microbubbles or nanobubbles.

### SUMMARY OF THE INVENTION

In the culture apparatuses disclosed in JP1976-038480A (JP-S51-038480A) and US2019/0316072A, the amount of the raw material gas to be supplied may be limited to ensure safety, and there is a concern that a raw material shortage may occur. On the other hand, in the culture apparatuses disclosed in JP2016-002040A and JP2018-117578A, a dissolution rate of the raw material gas is improved through reduction in bubble size, and thus it is possible to ensure the utilization efficiency and safety of the raw material gas while eliminating the raw material shortage.

However, in the culture apparatuses disclosed in JP2016-002040A and JP2018-117578A, there is a concern that the energy consumption required for reducing the size of bubbles is large and the culture apparatuses are contrary to the social trend of reducing greenhouse gas emissions.

The disclosed technology provides a culture apparatus capable of improving a dissolution rate of a raw material gas while suppressing energy consumption required for reducing the size of bubbles.

In order to achieve the above object, a culture apparatus according to a first aspect of the disclosed technology is a culture apparatus for culturing a microorganism or a cell that uses a raw material gas containing a combustible gas for proliferation or production of an organic substance, the culture apparatus comprising: a culture tank that contains a culture solution for culturing the microorganism or the cell; a circulation path that takes out the culture solution from an outlet port provided in the culture tank and returns the taken-out culture solution to the culture tank from a return port provided in the culture tank; a pump that generates a pressure for circulating the culture solution through the circulation path to generate, as a flow of the culture solution in the circulation path, a downward flow from an upper side toward a lower side in a vertical direction; and a bubble generating device that generates bubbles having a diameter of 100 µm or more to supply the raw material gas to the circulation path as bubbles, in which the outlet port is disposed above the return port in the vertical direction, and the bubble generating device is disposed on an upstream side of a midpoint of a length from the outlet port to the return port.

In the first aspect, it is preferable that, in a case where an average flow velocity of the downward flow of the culture solution in the circulation path is denoted by Vₚᵢₚₑ and a rising velocity of the bubbles is denoted by V_{B}, Vₚᵢₚₑ satisfies a condition of the following expression, ${V}_{pipe} > {V}_{B} .$

In the first aspect, it is preferable that, in a case where a height in the vertical direction from the return port to the bubble generating device in the circulation path is denoted by Hₚᵢₚₑ, a height from a bottom of the culture tank to a liquid surface of the culture solution is denoted by H_{L}, and a height from the bottom of the culture tank to the return port is denoted by Hᵢₙ, Vₚᵢₚₑ satisfies a condition of the following expression, ${V}_{pipe} < \left(1+\frac{{H}_{pipe}}{{V}_{B} ⋅ 1 1.2 \left[S\right] - {H}_{L} + {H}_{in}}\right) {V}_{B} .$

In the first aspect, it is preferable that the circulation path is a path dedicated to circulation and provided independently of a recovery path for recovering part of the microorganism or the cell from the culture solution.

In the first aspect, it is preferable that the return port is provided in a range of 1/4 or less of a height of a liquid surface from a bottom in the culture tank.

In the first aspect, it is preferable that the microorganism is hydrogen-oxidizing bacteria, and the raw material gas contains hydrogen as the combustible gas and contains oxygen and carbon dioxide as other components.

In the first aspect, it is preferable that the raw material gas contains hydrogen as the combustible gas and contains oxygen and carbon dioxide as other components, and each component of the hydrogen, the oxygen, and the carbon dioxide is separately supplied.

In the first aspect, it is preferable that the culture apparatus further comprises a compositional ratio control mechanism that measures a compositional ratio of a mixed gas in a space present above a liquid surface of the culture solution in the culture tank and supplies an adjustment gas into the space according to the measured compositional ratio of the mixed gas to maintain the compositional ratio of the mixed gas in the space at a target value outside a preset explosive range.

A culture apparatus according to a second aspect of the disclosed technology is a culture apparatus for culturing a microorganism or a cell that uses a raw material gas containing a combustible gas for proliferation or production of an organic substance, the culture apparatus comprising: a culture tank that contains a culture solution for culturing the microorganism or the cell; a circulation path that takes out the culture solution from an outlet port provided in the culture tank and returns the taken-out culture solution to the culture tank from a return port provided in the culture tank; a pump that generates a pressure for circulating the culture solution through the circulation path to generate, as a flow of the culture solution in the culture tank, a downward flow from an upper side toward a lower side in a vertical direction; and a bubble generating device that generates bubbles having a diameter of 100 µm or more to supply the raw material gas to the culture tank as bubbles.

In the second aspect, it is preferable that, in a case where a height of a liquid surface of the culture solution in the culture tank is denoted by H_{L}, an average flow velocity of the downward flow of the culture solution in the culture tank is denoted by Vₜₐₙₖ, and a rising velocity of the bubbles is denoted by V_{B}, Vₜₐₙₖ satisfies a condition of the following expression, $\frac{{H}_{L}}{{V}_{B} - {V}_{tank}} > 1 1 . 2 \left[S\right] .$

In the second aspect, it is preferable that the circulation path is a path dedicated to circulation and provided independently of a recovery path for recovering a part of the microorganism or the cell from the culture solution.

In the second aspect, it is preferable that the outlet port is disposed below the bubble generating device.

In the second aspect, it is preferable that the microorganism is hydrogen-oxidizing bacteria, and the raw material gas contains hydrogen as the combustible gas and contains oxygen and carbon dioxide as other components.

In the second aspect, it is preferable that the raw material gas contains hydrogen as the combustible gas and contains oxygen and carbon dioxide as other components, and the raw material gas is supplied such that each component of the hydrogen, the oxygen, and the carbon dioxide is separately supplied.

In the second aspect, it is preferable that the culture apparatus further comprises a compositional ratio control mechanism that measures a compositional ratio of a mixed gas in a space present above a liquid surface of the culture solution in the culture tank and supplies an adjustment gas into the space according to the measured compositional ratio of the mixed gas to maintain the compositional ratio of the mixed gas in the space at a target value outside a preset explosive range.

According to the disclosed technology, it is possible to improve a dissolution rate of the raw material gas while suppressing energy consumption required for reducing the size of the bubbles.

### BRIEF DESCRIPTION OF THE DRAWINGS

FIG. 1 is a diagram illustrating a function of a culture apparatus.
FIG. 2 is a diagram illustrating an overall configuration of the culture apparatus.
FIG. 3 is a diagram illustrating a configuration in which a raw material gas is supplied for each component.
FIG. 4 is a diagram illustrating a configuration in which a culture solution is circulated.
FIG. 5 is a diagram illustrating an example in which a circulation path extends in a vertical direction.
FIG. 6 is a diagram illustrating an example in which the circulation path is inclined with respect to the vertical direction.
FIG. 7 is a graph illustrating a relationship between a dissolved gas concentration and a residence time for complete dissolution.
FIG. 8 is a diagram illustrating a configuration of compositional ratio control.
FIG. 9 is a diagram illustrating an overall configuration of a culture apparatus of a second embodiment.
FIG. 10 is a diagram illustrating a configuration in which a culture solution of the second embodiment is circulated.

### DESCRIPTION OF THE PREFERRED EMBODIMENTS

### [First Embodiment]

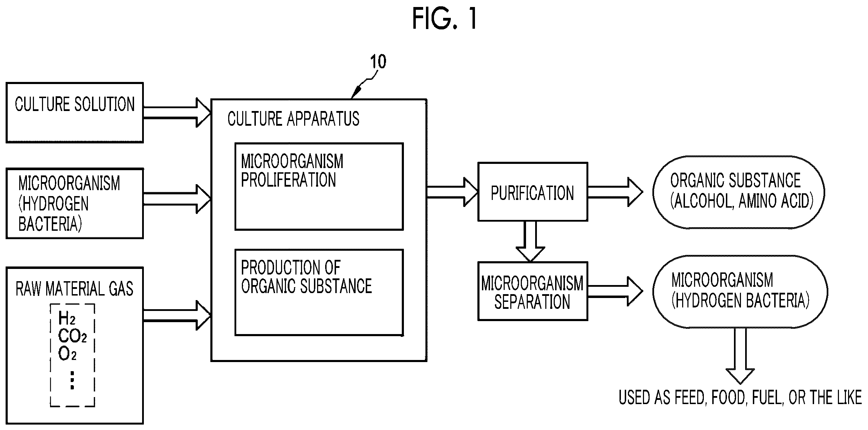

As illustrated in FIG. 1, a culture apparatus 10 of a first embodiment according to the disclosed technology cultures hydrogen-oxidizing bacteria, which are microorganisms, as an example of a culture target. A raw material for proliferating the hydrogen-oxidizing bacteria contains inorganic components such as ammonium sulfate, in addition to hydrogen (H₂), oxygen (O₂), and carbon dioxide (CO₂). Hydrogen is an example of a combustible gas. The culture apparatus 10 proliferates the hydrogen-oxidizing bacteria in a culture solution by supplying a raw material gas to the culture solution. In addition, in the culture solution, the hydrogen-oxidizing bacteria efficiently produce an organic substance such as alcohol and an amino acid by modifying a gene of the hydrogen-oxidizing bacteria. The culture solution is purified to extract the organic substance. The extracted organic substance is used for manufacturing, for example, a chemical product or a bioproduct. In addition, the hydrogen-oxidizing bacteria are separated from the culture solution, and it is being studied to use the separated hydrogen-oxidizing bacteria as, for example, a feed, a food, a fuel, or the like.

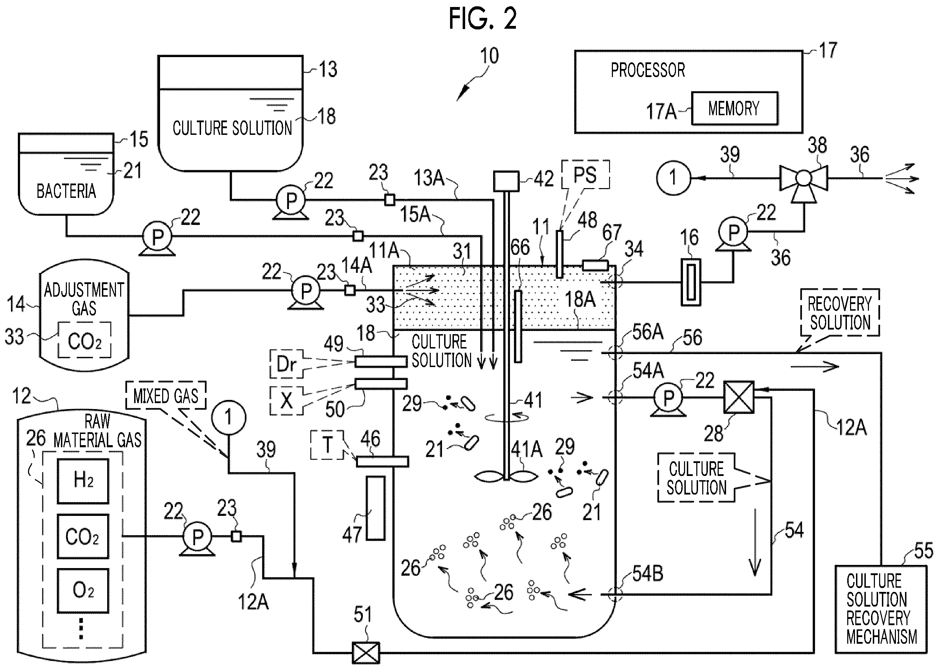

As illustrated in FIG. 2, the culture apparatus 10 comprises a culture tank 11, a raw material gas tank 12, a culture solution tank 13, an adjustment gas tank 14, a culture target tank 15, and a processor 17. The culture tank 11 contains a culture solution 18. Examples of the hydrogen-oxidizing bacteria 21 include bacteria such as bacteria of the genus Hydrogenophilus described in JP6528295B. In this case, as the culture solution 18, as an example, an aqueous solution in which ammonium sulfate ((NH₄)₂SO₄), potassium dihydrogen phosphate (KH₂PO₄), dipotassium hydrogen phosphate (K₂HPO₄), sodium chloride (NaCl), and the like are dissolved in water is used as described in JP6528295B. The culture tank 11 is an example of a "culture tank" according to the disclosed technology. Instead of or in addition to the raw material gas tank 12, a gas generating device that generates a raw material gas 26 may be provided.

Supply paths 12A, 13A, 14A, and 15A of the raw material gas tank 12, the culture solution tank 13, the adjustment gas tank 14, and the culture target tank 15 are connected to the culture tank 11. Each of the supply paths 12A, 13A, 14A, and 15A is composed of a pipe, a valve, and the like. In addition, a pump 22 and a flow meter 23 that measures a flow rate of a fluid flowing through each supply path are disposed in each of the supply paths 12A, 13A, 14A, and 15A.

In the first embodiment, the supply path 12A of the raw material gas tank 12 is connected to the culture tank 11 through a culture solution circulation path (hereinafter, simply referred to as a circulation path) 54 that circulates the culture solution 18. The raw material gas 26 is supplied from the supply path 12A to the culture solution 18 in the circulation path 54, and the culture solution 18 is supplied to the culture tank 11 by flowing into the culture tank 11 through the circulation path 54.

The culture solution tank 13 contains the culture solution 18. The culture solution 18 is supplied from the culture solution tank 13 to the culture tank 11 through the supply path 13A by driving the pump 22 on the supply path 13A. A supply timing and a supply amount of the culture solution 18 are controlled by the processor 17 that controls the driving of the pump 22.

The culture target tank 15 contains hydrogen-oxidizing bacteria 21, which are an example of a culture target. The hydrogen-oxidizing bacteria 21 are supplied from the culture target tank 15 to the culture tank 11 through the supply path 15A by driving the pump 22 on the supply path 15A. A supply timing and a supply amount of the hydrogen-oxidizing bacteria 21 are controlled by the processor 17 that controls the driving of the pump 22.

The raw material gas tank 12 contains the raw material gas 26. As described above, the raw material gas 26 is, for example, a gas containing each component such as hydrogen (H₂), oxygen (O₂), and carbon dioxide (CO₂). The raw material gas 26 is supplied from the raw material gas tank 12 to the culture tank 11 through the supply path 12A by driving the pump 22 on the supply path 12A. A supply timing and a supply amount of the raw material gas 26 are controlled by the processor 17 that controls the driving of the pump 22.

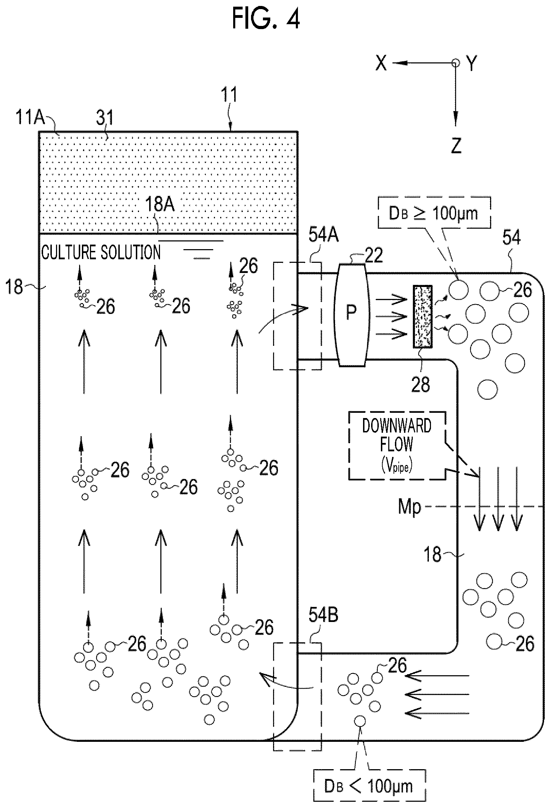

A downstream end of the supply path 12A is connected to the circulation path 54. More specifically, the sparger 28 is disposed in the circulation path 54, and the downstream end of the supply path 12A is connected to the sparger 28. The sparger 28 is used to disperse and supply the raw material gas 26 into the culture solution 18. As the sparger 28, for example, a ventilation tube formed of a porous material is used. The porous material of the sparger 28 is preferably made of sintered metal or ceramics. The sparger 28 is a bubble generating device that generates the raw material gas 26 as bubbles in the culture solution 18. The sparger 28 generates bubbles having a diameter of 100 µm or more. That is, in a case where a bubble diameter which is a diameter of the bubbles of the raw material gas 26 is denoted by D_{B}, the sparger 28 generates the raw material gas 26 having D_{B} of 100 µm or more (that is, D_{B} ≥ 100 µm) (see FIG. 4). The sparger 28 is an example of a "bubble generating device" that supplies the raw material gas 26 to the circulation path 54 as bubbles according to the disclosed technology.

In addition, the bubble generating device has various methods such as a swirl flow type that generates bubbles by a high-speed swirl flow, an ejector type (also referred to as a Venturi type) that generates bubbles by a rapid pressure change in a gas-liquid flow path, and a static mixer type that generates bubbles by a shearing force of an obstacle in the gas-liquid flow path, in addition to a fine pore type such as the sparger 28. As the bubble generating device, a type other than the sparger 28 may be used.

The circulation path 54 is provided on an outer peripheral surface of the culture tank 11 (see also FIG. 4). The circulation path 54 takes out the culture solution 18 from an outlet port 54A provided in the culture tank 11 and returns the taken-out culture solution 18 to the culture tank 11 from a return port 54B provided in the culture tank, thereby circulating the culture solution 18 in the culture tank 11. In the circulation path 54 of the first embodiment, the outlet port 54A is disposed above the return port 54B in the vertical direction (corresponding to the Z direction in FIG. 4). The pump 22 is also provided in the circulation path 54. The pump 22 is a pump that generates a pressure for circulating the culture solution 18 through the circulation path 54, and generates a downward flow from an upper side to a lower side in the vertical direction as a flow of the culture solution 18 in the circulation path 54.

In addition, in the circulation path 54, the sparger 28 is disposed on an upstream side of a midpoint Mp (see FIG. 4) of a length from the outlet port 54A to the return port 54B of the circulation path 54. The pump 22 is disposed on the upstream side of the sparger 28. Since the bubbles generated by the sparger 28 rise to the upper side in the vertical direction in a state where the culture solution 18 is stationary, the bubbles are pushed and flowed toward the return port 54B by the downward flow of the culture solution 18 generated by the pump 22. As a result, the bubbles of the raw material gas 26 flow into the culture tank 11.

A part of the raw material gas 26 supplied into the culture solution 18 is dissolved in the culture solution 18. The dissolved raw material gas 26 is taken up by the hydrogen-oxidizing bacteria 21 and is consumed for the proliferation of the hydrogen-oxidizing bacteria 21 or the production of the organic substance 29. The organic substance 29 is, for example, an alcohol or an amino acid. The raw material gas 26 that is not dissolved in the culture solution 18 is released into a space 11A present above a liquid surface 18A of the culture solution 18. The space 11A is filled with a mixed gas 31 which is the raw material gas 26 released without being dissolved in the culture solution 18.

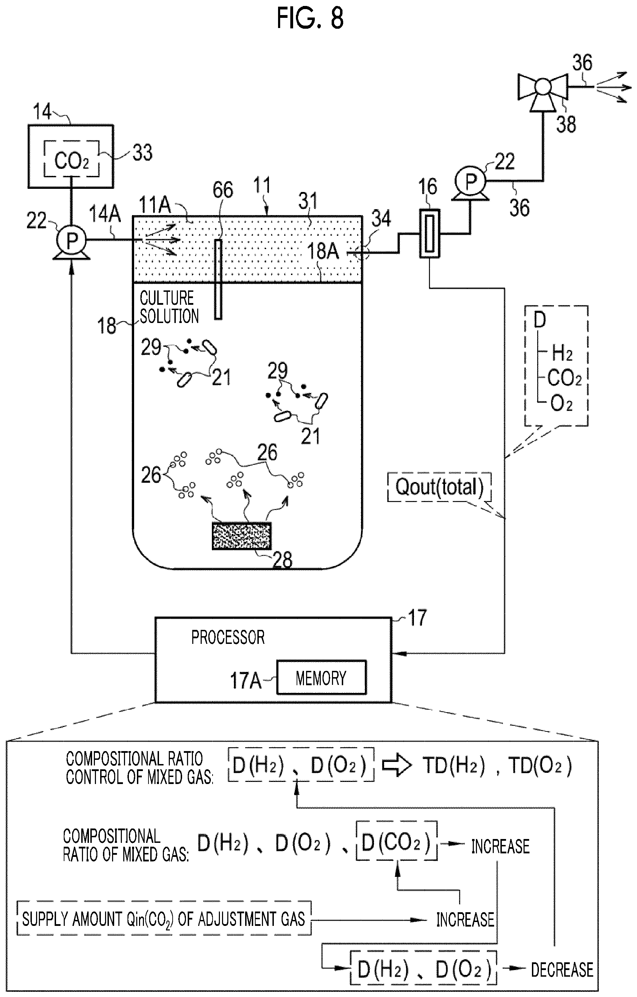

The adjustment gas tank 14 contains an adjustment gas 33. The adjustment gas 33 is supplied from the adjustment gas tank 14 to the space 11A of the culture tank 11 through a supply path 14A by driving the pump 22 on the supply path 14A. A supply timing and a supply amount of the adjustment gas 33 are controlled by the processor 17 that controls the driving of the pump 22. The mixed gas 31 in the space 11A contains hydrogen and oxygen which are components of the raw material gas 26. The hydrogen is a combustible gas. The adjustment gas 33 is used to maintain a compositional ratio of the mixed gas 31 in the space 11A at a target value outside a preset explosive range. The compositional ratio control will be described later (see FIG. 8).

In addition, the mixed gas 31 contains the components of the raw material gas 26. Therefore, a part of the mixed gas 31 can be returned to the culture tank 11 through a gas circulation path 39 and can be reused as the raw material gas 26. The gas circulation path 39 is connected to the supply path 12A of the raw material gas 26. The mixed gas 31 that is reused as the raw material gas 26 is also supplied to the culture tank 11 through the sparger 28 provided in the circulation path 54. The mixed gas 31 that is not reused is discharged. Although not shown, in a case where the mixed gas 31 that is not reused is discharged, the mixed gas 31 is diluted with an inert gas such as carbon dioxide and then is released to the atmosphere.

A discharge path 36 for drawing out the mixed gas 31 is connected to the culture tank 11, and the pump 22 and a three-way valve 38 are provided in the discharge path 36. The pump 22 generates a pressure for discharging and circulating the mixed gas 31. The three-way valve 38 switches between a state where the discharge path 36 communicates with the gas circulation path 39 and a state where the communication between the discharge path 36 and the gas circulation path 39 is closed. The three-way valve 38 switches whether to send the mixed gas 31 to the gas circulation path 39 or to exhaust the mixed gas 31 to the outside. In addition, a compositional ratio measuring unit 16 that measures a compositional ratio of the mixed gas 31 is provided in the discharge path 36. The compositional ratio measuring unit 16 is used for compositional ratio control of the mixed gas 31, which will be described later.

### (Supply of Each Component of Raw Material Gas)

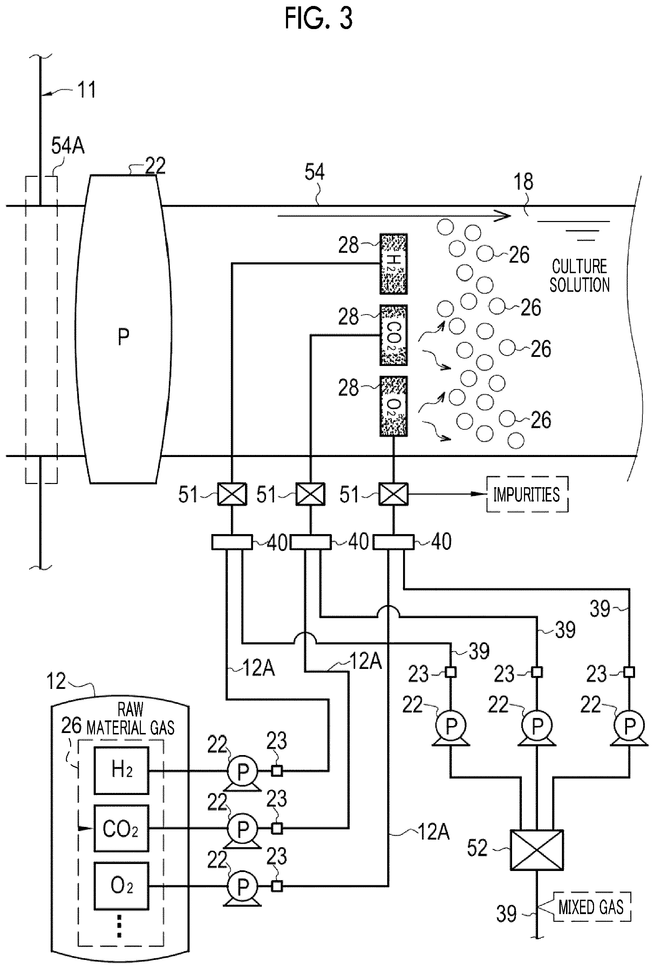

In addition, in FIG. 2, for convenience, the supply path 12A of the raw material gas 26 and the gas circulation path 39 of the mixed gas 31 are shown as one path in order to suppress the complication of the drawing. However, in reality, as shown in FIG. 3, the supply path 12A and the gas circulation path 39 are separated for each component of hydrogen, oxygen, carbon dioxide, and the like, and a plurality of components can be individually supplied. The pump 22 and the flow meter 23 are provided in each of the supply path 12A and the gas circulation path 39 for each component. In addition, the sparger 28 is also provided for each component of hydrogen (H₂), oxygen (O₂), carbon dioxide (CO₂), and the like. Each of the supply path 12A and the gas circulation path 39 for each component is connected to each of the spargers 28 corresponding to each component.

### (Removal of Impurities from Raw Material Gas)

A connecting member 40 connects the supply path 12A and the gas circulation path 39 on the upstream side of the sparger 28 for each component. As a result, the supply path 12A and the gas circulation path 39 are integrated into one path and are connected to the sparger 28. An impurity removal mechanism 51 is provided between each connecting member 40 and each sparger 28. The impurity removal mechanism 51 removes impurities from the raw material gas 26 supplied to the culture tank 11. The impurities include sulfur oxides or nitrogen oxides (NO, NO₂, and the like). The impurity removal mechanism 51 is a mechanism that uses any one of a membrane separation method, a cryogenic separation method, a physical adsorption method, or a chemical absorption method as a method of separating impurities from the raw material gas 26.

### (Separation of Components Included in Mixed Gas)

In addition, since the mixed gas 31 is a mixture of each component of hydrogen, oxygen, carbon dioxide, and the like, the gas circulation path 39 is provided with a component separation mechanism 52 that separates the respective components included in the mixed gas 31. The component separation mechanism 52 separates the respective components included in the mixed gas 31, and each separated component is supplied to each gas circulation path 39 provided for each component. The component separation mechanism 52 is a mechanism that uses any one of a membrane separation method, a cryogenic separation method, a physical adsorption method, or a chemical absorption method as a method of separating each component from the mixed gas 31.

As described above, in a case where the raw material gas 26 is supplied to the culture tank 11, individually supplying each component of hydrogen (H₂), oxygen (O₂), carbon dioxide (CO₂), and the like has an advantage in terms of culture efficiency and safety. The advantage of the culture efficiency is that the raw material gas 26 supplied to the culture tank 11 is easily adjusted to an appropriate composition from the viewpoint of the culture efficiency. In addition, the advantage of the safety is as follows. That is, the three elements of the explosion are a combustible gas, oxygen, and an ignition source, but before the raw material gas 26 is supplied to the culture tank 11, it is possible to separate and handle hydrogen and oxygen, which are the combustible gas among the three elements of the explosion, and the risk of explosion can be further reduced.

In addition, the component separation mechanism 52 may separate components such as nitrogen and methane as components included in the mixed gas 31 in addition to hydrogen, oxygen, and carbon dioxide. Nitrogen, methane, and the like have an action of reducing the solubility of the raw material gas 26 in the culture solution 18, and thus, by separating these components when the mixed gas 31 is reused, the solubility of the raw material gas 26 can be improved.

### (Recovery of Culture Solution)

In addition, as illustrated in FIG. 2, a culture solution recovery path 56 is connected to the culture tank 11. One end of the culture solution recovery path 56 is connected to a recovery solution outlet port 56A provided in the culture tank 11, and the other end is connected to a culture solution recovery mechanism 55. The culture solution recovery path 56 takes out a part of the culture solution 18 from the culture tank 11 as a recovery solution. The culture solution recovery mechanism 55 recovers the culture solution 18 containing the organic substance 29 from the culture solution 18 taken out from the culture tank 11 through the culture solution recovery path 56. The culture solution recovery mechanism 55 includes a recovery solution tank that contains a recovery solution, which is the recovered culture solution 18, a separation mechanism that separates a purification target from the recovery solution, and the like. The recovery solution is transferred to a purification apparatus (not shown), and the organic substance 29 is extracted from the recovery solution. In addition, the remaining culture solution 18 after being recovered by the culture solution recovery mechanism 55, which is the culture solution 18 containing the hydrogen-oxidizing bacteria 21, may be returned to the culture tank 11. In a case where the proliferated hydrogen-oxidizing bacteria 21 are purified, the hydrogen-oxidizing bacteria 21 are recovered by a method different from that of the organic substance 29, for example.

### (Stirring of Culture Tank, Temperature Adjustment, and Sensing of Various Types of Information)

In addition, the culture tank 11 is provided with a stirring rod 41 that stirs the culture solution 18. The stirring rod 41 is provided with a screw 41A on a support rod, and the screw 41A is provided to be disposed in the culture solution 18. The stirring rod 41 rotates about the support rod as a rotation axis by driving of a motor 42 controlled by the processor 17. As a result, the culture solution 18 is stirred. The stirring rod 41 diffuses the hydrogen-oxidizing bacteria 21 and the raw material gas 26 in the culture solution 18 so that a local bias does not occur by stirring the culture solution 18.

In addition, the culture tank 11 is provided with a thermometer 46, a temperature controller 47, a pressure gauge 48, a dissolved gas concentration measuring unit 49, and a cell density measuring unit 50. The thermometer 46 measures a temperature T in the culture solution 18. The temperature controller 47 heats or cools the culture solution 18 in the culture tank 11. The pressure gauge 48 measures a pressure PS in the space 11A. The dissolved gas concentration measuring unit 49 measures a dissolved gas concentration Dr of the raw material gas 26 dissolved in the culture solution 18.

The culture solution 18 is maintained at a target temperature suitable for culturing the hydrogen-oxidizing bacteria 21. The target temperature is, for example, 40°C or higher and 95°C or lower. The target temperature is set for each microorganism. In a case where the hydrogen-oxidizing bacteria 21 are the genus Hydrogenophilus, the target temperature is more preferably about 50°C. The culture solution 18 is heated to the target temperature, and may also be cooled in some cases. This is because the microorganism to be cultured may generate heat during proliferation and the temperature of the culture solution 18 may exceed the target temperature. The processor 17 controls the driving of the temperature controller 47 such that the temperature T of the culture solution 18 is maintained at the target temperature based on the temperature T measured by the thermometer 46.

In addition, the processor 17 controls the pressure of the mixed gas 31 in the space 11A based on the pressure PS measured by the pressure gauge 48. By setting the pressure PS of the mixed gas 31 to a target pressure, the solubility of the raw material gas 26 in the culture solution 18 is improved. As described above, the processor 17 ensures appropriate solubility in the culture solution 18 by controlling the pressure of the mixed gas 31. As a result, the culture efficiency of the hydrogen-oxidizing bacteria 21 is improved. The adjustment target of the pressure PS of the mixed gas 31 is at least one of a supply amount of the raw material gas 26, a discharge amount of the mixed gas 31, and a supply amount of the adjustment gas 33. The processor 17 adjusts the supply amount or the discharge amount of these gases by controlling the driving of each pump 22.

More specifically, the dissolved gas concentration measuring unit 49 measures the concentration of each component (hydrogen, oxygen, and carbon dioxide) contained in the raw material gas 26 dissolved in the culture solution 18. The dissolved gas concentration measuring unit 49 is composed of, for example, a dissolved hydrogen meter (for example, model number KM2100DH, manufactured by Kyoei Electronic Laboratory Co., Ltd.) that measures a dissolved hydrogen concentration (Dr(H₂)), a dissolved oxygen meter (for example, model number InPro6860i, manufactured by METTLER TOLEDO) that measures a dissolved oxygen concentration (Dr(O₂)), and a dissolved carbon dioxide meter (for example, model number InPro5000i, manufactured by METTLER TOLEDO) that measures a dissolved carbon dioxide concentration (Dr(CO₂)). The dissolved gas concentration measuring unit 49 outputs the measured value to the processor 17.

As shown in FIG. 3, the raw material gas tank 12 contains each component (hydrogen, oxygen, and carbon dioxide) of the raw material gas 26. In addition, the supply path 12A and the pump 22 are provided for each component.

The processor 17 acquires the measured value of the dissolved gas concentration for each component from the dissolved gas concentration measuring unit 49, compares the dissolved gas concentration with the target value for each component, and supplies the component that is insufficient in the raw material gas 26 to the culture tank 11. The processor 17 can maintain the dissolved gas concentration of the raw material gas 26 in the culture solution 18 at an appropriate value by controlling the supply amount of each component of the raw material gas 26.

The cell density measuring unit 50 is an example of a density measuring unit that measures the density of the culture target present in the culture solution 18 in the culture tank 11. The cell density measuring unit 50 is composed of, for example, a total cell density sensor (for example, model number Dencytee RS485, manufactured by HAMILTON). In the present example, since the culture target is the hydrogen-oxidizing bacteria 21, the cell density measuring unit 50 measures a cell density X which is the density of the hydrogen-oxidizing bacteria 21 in the culture solution 18. The cell density X is the mass or the number of cells (in the present example, the hydrogen-oxidizing bacteria 21) contained in the unit volume of the culture solution 18. The cell density X has a positive correlation with the consumption amount of the raw material gas 26 consumed by the hydrogen-oxidizing bacteria 21. By measuring the cell density X, for example, the consumption amount of the raw material gas 26 consumed by the hydrogen-oxidizing bacteria 21 can be calculated, or a process check for verifying whether the consumption amount of the raw material gas 26 is appropriate with respect to the amount of the hydrogen-oxidizing bacteria 21 can be performed.

The liquid level gauge 66 measures a liquid level of the culture solution 18 in the culture tank 11, that is, the height of the liquid surface 18A. The amount of the culture solution 18 present in the culture tank 11 can be grasped by the liquid level of the culture solution 18 measured by the liquid level gauge 66. The liquid level gauge 66 is used for a process check of whether the amount of the culture solution 18 is appropriate, calculation of a required replenishment amount of the culture solution 18, and the like.

In addition, the culture tank 11 is provided with an explosion vent 67. The explosion vent 67 is a configuration for safely releasing the pressure in the culture tank 11 by being destroyed earlier than other parts in a case where an explosion occurs in the culture tank 11, thereby suppressing the expansion of damage. A place where no large damage occurs even in a case where the pressure is released, or a place where a measure is taken to prevent the damage from expanding is selected in the vicinity of the explosion vent 67.

The processor 17 is configured with, for example, a central processing unit (CPU) and a random access memory (RAM), and controls each unit of the culture apparatus 10 as described above. In addition, the memory 17A is a non-volatile memory in which various types of setting information are stored, such as a flash memory.

The operation of the above-described configuration will be described with reference to FIG. 4. FIG. 4 shows a state in which the bubble diameter of the raw material gas 26 supplied in the culture solution 18 circulating through the culture tank 11 and the circulation path 54 changes. In FIG. 4, for convenience, only those necessary for describing the circulation of the culture solution 18 are shown, and other configurations are omitted in order to suppress the complication of the drawing. As described above, the sparger 28 is disposed on the upstream side of the midpoint Mp of the circulation path 54, and generates the raw material gas 26 having a bubble diameter of 100 µm or more (that is, D_{B} ≥ 100 µm).

Since the bubbles of the raw material gas 26 have a smaller specific gravity than the culture solution 18, the bubbles tend to rise upward in the vertical direction (Z direction in FIG. 4) in a case where there is no flow of the culture solution 18. However, the pump 22 generates the downward flow of the culture solution 18 in the circulation path 54. The bubbles of the raw material gas 26 are pushed by the downward flow and move in the circulation path 54 toward the return port 54B. The dissolution of the raw material gas 26 progresses as the residence time in the culture solution 18 is longer, and the bubble diameter gradually decreases as the dissolution progresses, and disappears when the dissolution is complete. In the example shown in FIG. 4, the bubble diameter is 100 µm or more immediately after the bubbles are generated by the sparger 28, but as the residence time of the culture solution 18 increases, the bubble diameter decreases, and the bubble diameter is, for example, less than 100 µm in the vicinity of the return port 54B of the circulation path 54. In this way, the raw material gas 26 flows into the culture tank 11 from the return port 54B in a state where the bubble diameter is small.

The bubbles of the raw material gas 26 that have flowed into the culture tank 11 rise toward the liquid surface 18A in the culture tank 11. Even during the rising, the dissolution of the bubbles in the culture solution 18 progresses, and the bubbles become smaller as the bubbles approach the liquid surface 18A. The smaller the bubble diameter, the slower the rising velocity. Therefore, the smaller the bubble diameter at the time of flowing into the culture tank 11, the slower the rising velocity to the liquid surface 18A in the culture tank 11, so that the residence time in the culture solution 18 is longer than that in a case where the bubble diameter is large. In addition, since the dissolution also progresses as the bubble diameter is smaller, the dissolution rate of the raw material gas 26 in the culture solution 18 is improved by causing the raw material gas 26 in a state where the bubble diameter is small to flow into the culture tank 11.

As described above, the culture apparatus 10 includes the circulation path 54 that takes out the culture solution 18 from the outlet port 54A provided in the culture tank 11 and returns the taken-out culture solution 18 to the culture tank 11 from the return port 54B provided in the culture tank 11, the pump 22 that generates a pressure for circulating the culture solution 18 through the circulation path 54 to generate, as a flow of the culture solution 18 in the circulation path 54**,** a downward flow from an upper side toward a lower side in the vertical direction, and the sparger 28 which is an example of a bubble generating device. The sparger 28 generates bubbles having a diameter of 100 µm or more, and supplies the raw material gas 26 to the circulation path 54 as bubbles. The outlet port 54A is disposed above the return port 54B in the vertical direction, and the sparger 28 is disposed on the upstream side of the midpoint Mp of the length from the outlet port 54A to the return port 54B. Therefore, it is possible to improve the dissolution rate of the raw material gas 26 while suppressing the energy consumption required for reducing the size of the bubbles.

That is, in the circulation path 54, the culture apparatus 10 generates the bubbles of the raw material gas 26 on the upstream side and generates the downward flow in a direction opposite to the rising direction of the bubbles as the flow of the culture solution 18, thereby causing the bubbles to flow into the culture tank 11 from the return port 54B while suppressing the backflow of the bubbles toward the outlet port 54A. The dissolution of the bubbles in the culture solution 18 progresses while the bubbles move from the upstream side to the return port 54B in the circulation path 54. Therefore, at the time of flowing into the culture tank 11 from the return port 54B, for example, reduction in the size of the bubbles proceeds as compared with a configuration in which the same performance sparger 28 is disposed at the bottom in the culture tank 11. Since the smaller the bubbles, the lower the rising velocity, the residence time in the culture tank (that is, the rising time to the liquid surface 18A in the culture tank 11) is longer. As a result, the dissolution rate of the raw material gas is improved.

In addition, as a method for reducing the size of the bubbles, there is a method of using a bubble generating device that generates microbubbles or nanobubbles having a bubble diameter of less than 100 µm as shown in the related art. However, the culture apparatus 10 does not use a bubble generating device that generates microbubbles or nanobubbles, and uses a bubble generating device that generates bubbles having a diameter of 100 µm or more. In addition, the culture apparatus 10 extends the residence time of the bubbles by devising the disposition of the circulation path and the bubble generating device.

As a result, the culture apparatus 10 obtains the above-described effect that the dissolution rate of the raw material gas 26 can be improved while suppressing the energy consumption required for reducing the size of the bubbles, as compared with a case where a bubble generating device that generates microbubbles or nanobubbles is used.

In a case where the dissolution rate of the raw material gas is improved, the culture efficiency is improved, and in addition, the undissolved combustible raw material gas 26 released into the space 11A as the mixed gas 31 is suppressed, so that the safety is also improved. Here, the dissolution rate of the raw material gas 26 is defined as follows. In a case where the supply amount of the raw material gas to the culture tank is denoted by Qin, the discharge amount from the culture tank 11 is denoted by Qout, and the dissolution rate of the raw material gas is denoted by R (%), R (%) = (1 - Qout/Qin) × 100.

In such a culture apparatus 10, it is preferable that the processor 17 controls the flow velocity of the downward flow of the culture solution 18 flowing in the circulation path 54 as follows. That is, in a case where the average flow velocity of the downward flow of the culture solution 18 in the circulation path 54 is denoted by Vₚᵢₚₑ and the rising velocity of the bubbles is denoted by V_{B}, it is preferable to control Vₚᵢₚₑ to satisfy a condition of Expression (1).${V}_{pipe} > {V}_{B}$ (1) ${V}_{B} = \frac{{D}_{B}^{2} {ρ}_{L} g}{1 8 η}$ (2)

Here, in Expression (2), D_{B} is the diameter of the bubbles of the raw material gas 26, ρ_{L} is the culture solution density, g is the acceleration due to gravity, and η is the viscosity of the culture solution 18. The culture solution density ρ_{L} is the mass of the culture solution 18 per unit volume, and is different from the cell density X described above. The culture solution density ρ_{L} is measured by a specific gravity meter (for example, a portable density/specific gravity meter DA-130N manufactured by Kyoto Electronics Manufacturing Co., Ltd.). The culture solution density ρ_{L} does not significantly fluctuate even in a case where the cell density X changes. Therefore, in the calculation of Expression (2), a value measured in advance by the specific gravity meter is used as the culture solution density ρ_{L}. That is, the rising velocity V_{B} of the bubbles can be calculated in advance before the culture is started by using Expression (2) in a case where the culture solution 18 to be used and the bubble diameter D_{B} generated by the sparger 28 are known.

Further, in a case where there is a distribution of the bubble diameter in the circulation path 54, D_{B} in Expression (2) is the average volumetric bubble diameter. In a case where there are N bubbles and the diameter of the i-th bubble is denoted by dᵢ, the average volumetric bubble diameter D_{B} is represented by the following Expression (3).${D}_{B} = \frac{1}{N} {\left({\sum }_{i = 1}^{N} {d}_{i}^{3}\right)}^{\frac{1}{3}}$ (3)

As shown in Expression (1), by setting the average flow velocity Vₚᵢₚₑ of the downward flow to be faster than the rising velocity V_{B} of the bubbles, it is possible to more reliably suppress the bubbles from flowing back with respect to the circulation direction of the culture solution 18. Therefore, the bubbles that attempt to rise in the circulation path 54 are reliably pushed and flowed toward the return port 54B, and thus the supply amount of the raw material gas supplied to the culture tank 11 can be appropriately maintained.

The processor 17 controls the pump 22 that generates the downward flow (see also FIG. 4) in the circulation path 54 to control Vₚᵢₚₑ of the downward flow. In the memory 17A, the output of the pump 22 is set such that Vₚᵢₚₑ satisfying Expression (1) is obtained according to the characteristics of the culture solution 18 and the dimensions of the culture tank 11 and the circulation path 54. The processor 17 controls the output of the pump 22 according to the set value to control Vₚᵢₚₑ of the downward flow.

Regarding the bubble diameter D_{B} in the culture solution 18, the initial diameter of the bubbles generated by the sparger 28 is determined according to the performance of the sparger 28. In a case where the rate of change in which the diameter of the bubbles is reduced is measured in advance according to the dimensions of the circulation path 54 to be used, the average diameter of the bubbles in the culture solution 18 can be derived. The diameter of the bubbles can be measured by, for example, a laser diffraction/scattering method, a dynamic image analysis method, or the like. As a specific measuring device, for example, a focused beam reflectance measurement device ParticleTrack G400 or an inline microscope EasyViewer400 manufactured by Mettler-Toledo International Inc. can be used.

In addition, the culture solution density ρ_{L} fluctuates during the culture, but as described above, the measured value measured in advance is used as the culture solution density ρ_{L}, and Vₚᵢₚₑ is controlled based on the culture solution density ρ_{L} of the culture solution 18 measured in advance. In a case where the fluctuation of the culture solution density ρ_{L} during the culture is taken into consideration, the following may be performed. That is, for example, the correlation between the cell density X and the culture solution density ρ_{L} is measured in advance, and the correlation is stored in the memory 17A. Then, the processor 17 calculates the culture solution density ρ_{L} during the culture by using the measured value of the cell density X measured by the cell density measuring unit 50 during the culture and the correlation in the memory 17A. The processor 17 controls Vₚᵢₚₑ by using the calculated culture solution density ρ_{L}.

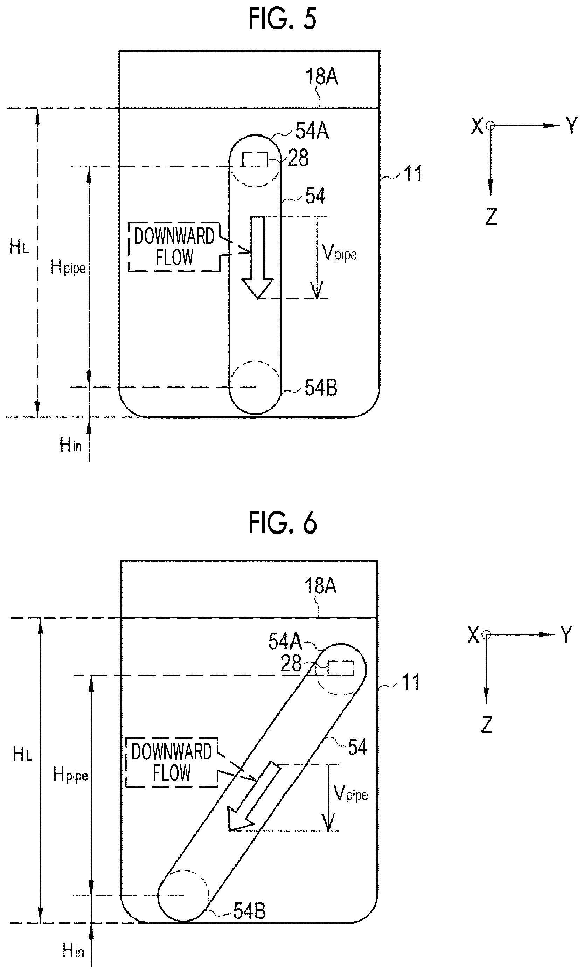

As shown in FIG. 5, the circulation path 54 may extend parallel to the vertical direction, or as shown in FIG. 6, the circulation path 54 may be inclined with respect to the vertical direction. As shown in FIGS. 5 and 6, the average flow velocity Vₚᵢₚₑ is the magnitude of the vertical component along the vertical direction.

The circulation path 54 may have a shape of a curve (including a spiral) in addition to a linear shape, in whole or in part. By adopting such a shape, the circulation path 54 can be flexibly disposed according to the space around the culture tank 11. In addition, by adopting a helical shape, a longer circulation path 54 can be disposed in the same space. By increasing the length of the circulation path 54, the residence time of the bubbles can be increased.

Further, it is preferable that the average flow velocity Vₚᵢₚₑ satisfies a condition of the following Expression (4). ${V}_{pipe} < \left(1+\frac{{H}_{pipe}}{{V}_{B} ⋅ 1 1 . 2 \left[S\right] - {H}_{L} + {H}_{in}}\right) {V}_{B}$ (4)

In Expression (4), Hₚᵢₚₑ is a height in the vertical direction from the center of the return port 54B in the circulation path 54 to the lower end of the sparger 28, as shown in FIGS. 5 and 6. H_{L} is a height from the bottom of the culture tank 11 to the liquid surface 18A. More specifically, H_{L} is an average height of the liquid surface 18A of the culture solution 18 in a case where the culture solution 18 is circulated during the culture. Hᵢₙ is a height from the bottom of the culture tank 11 to the center of the return port 54B. V_{B} is the rising velocity of the bubbles as described above.

Expression (4) is a condition for defining the upper limit of the average flow velocity Vₚᵢₚₑ. In a case where the average flow velocity Vₚᵢₚₑ is too fast, the residence time of the bubbles is shortened. In a case where the average flow velocity Vₚᵢₚₑ satisfies the condition of Expression (4), it is possible to secure a time required for practical use as the residence time of the bubbles in the culture tank 11.

Expression (4) is derived as follows. In a case where the residence time until the bubbles supplied from the sparger 28 reach the liquid surface 18A is denoted by T and the minimum time required for completely dissolving the bubbles in the culture solution 18 is denoted by Tₘᵢₙ, it is necessary to make T longer than Tₘᵢₙ. That is, it is necessary to satisfy a condition of T > Tₘᵢₙ.

T is the sum of the residence time of the bubbles in the culture tank 11 and the residence time in the circulation path 54. The residence time of the bubbles in the culture tank 11 is a value obtained by dividing the distance over which the bubbles rise in the culture tank 11 by the rising velocity, and the residence time in the circulation path 54 is a value obtained by dividing the substantial flow velocity of the bubbles in the circulation path 54 by the distance in the vertical direction in which the bubbles travel in the circulation path 54. Therefore, T > Tₘᵢₙ can be represented by the following Expression (5). In a case where Expression (5) is modified, Expression (6) is obtained, and in a case where 11.2 [s] is substituted into Tₘᵢₙ of Expression (6), Expression (4) is obtained.$T = \frac{{H}_{L} - {H}_{in}}{{V}_{B}} + \frac{{H}_{pipe}}{{V}_{pipe} - {V}_{B}} > {T}_{min}$ (5) ${V}_{pipe} < \left(1+\frac{{H}_{pipe}}{{V}_{B} {T}_{min} - {H}_{L} + {H}_{in}}\right) {V}_{B}$ (6)

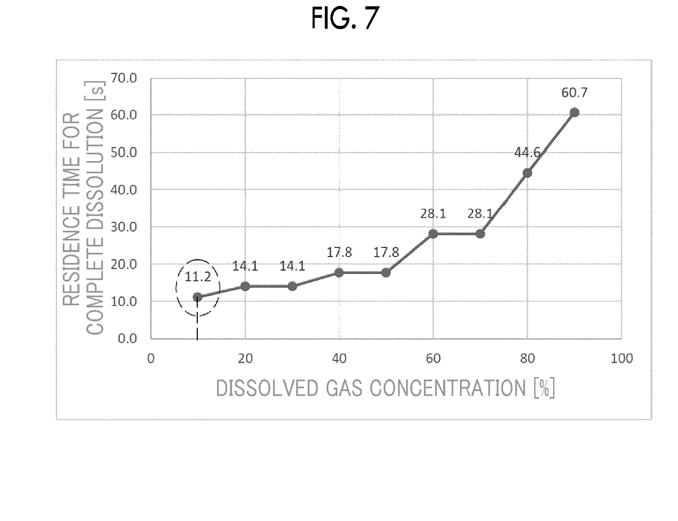

The time of 11.2 seconds in Expression (4) is a time derived from the experimental results shown in FIG. 7. FIG. 7 is a graph showing a relationship between the dissolved gas concentration and the residence time for complete dissolution. The horizontal axis is the dissolved gas concentration, and the dissolved gas concentration is the concentration of the raw material gas 26 dissolved in the culture solution 18.

The experiment was performed by the following method. Deaerated water was prepared in a cylindrical tank having a sufficient height, and a mixed gas of hydrogen, oxygen, and carbon dioxide (hydrogen:oxygen:carbon dioxide = 55:15:12 in ratio) was aerated from a sparger provided at a bottom of the tank until a predetermined dissolved gas concentration was reached. The dissolved gas concentration was measured using a dissolved gas concentration meter provided in the cylindrical layer, the dissolved gas concentration meter including a dissolved hydrogen meter (model number KM2100DH, manufactured by Kyoei Electronic Laboratory Co., Ltd.) for measuring a dissolved hydrogen concentration (Dr(H₂)), a dissolved oxygen meter (model number InPro6860i, manufactured by METTLER TOLEDO) for measuring a dissolved oxygen concentration (Dr(O₂)), and a dissolved carbon dioxide meter (model number InPro5000i, manufactured by METTLER TOLEDO) for measuring a dissolved carbon dioxide concentration (Dr(CO₂)). Thereafter, a small amount of bubbles were supplied from the sparger, and a maximum height Hₘₐₓ reached by the bubbles without being completely dissolved was recorded. The bubble diameter D_{B} from the sparger was calculated by measurement with a bubble diameter sensor (focused beam reflectance measurement device ParticleTrack G400 manufactured by Mettler-Toledo International Inc.) and the method of obtaining the average volumetric bubble diameter described above, and the rising velocity V_{B} of the bubbles was calculated. At this time, the residence time of the bubbles is calculated by Hₘₐₓ/V_{B}. The experiment was performed using spargers having different bubble diameters, and the shortest residence time of the bubbles was set as the residence time required for complete dissolution. The residence time required for complete dissolution was plotted with respect to the dissolved gas concentration, and the experimental results shown in FIG. 7 were obtained.

The residence time for complete dissolution is a residence time required for the raw material gas 26 to be completely dissolved in the culture solution 18 from the state of the bubbles, and is Tₘᵢₙ in Expression (5). In the experimental results shown in FIG. 7, the residence time for complete dissolution is 11.2 seconds in a case where the dissolved gas concentration is 10%. The residence time for complete dissolution increases as the dissolved gas concentration increases beyond 10%. The dissolved gas concentration is naturally varied depending on various conditions such as the consumption amount of the bacterial cells, but in a case where the residence time for completely dissolving the dissolved gas is secured in a case where the dissolved gas concentration is 10%, the minimum required dissolution rate of the raw material gas can be secured. The time of 11.2 seconds is the minimum time required for approaching the complete dissolution of the raw material gas 26 in practice.

In addition, the bubbles that have flowed into the culture tank 11 from the circulation path 54 decrease in size in the process of rising in the culture tank 11. However, since the change in the diameter of the bubbles is also complicated, the rising velocity V_{B} of the bubbles is calculated using the diameter of the bubbles at the time of flowing into the culture tank 11 from the circulation path 54 as a representative value. As described above, since the initial diameter of the bubbles generated by the sparger 28 is known, the diameter of the bubbles at the time of flowing into the culture tank 11 can be derived based on the initial diameter.

In addition, it is preferable that the return port 54B is provided in a range of 1/4 or less of the height of the liquid surface 18A from the bottom in the culture tank 11. As described above, the residence time of the bubbles of the raw material gas 26 in the culture tank 11 can be made longer as the distance to the liquid surface 18A is longer. By disposing the height of the return port 54B through which the bubbles of the raw material gas 26 flow into the culture tank 11 at a low position close to the bottom of the culture tank 11, the distance to the liquid surface 18A can be increased.

In addition, in the above-described embodiment, the circulation path 54 is a path dedicated to circulation and provided independently of the recovery path 56 for recovering a part of the hydrogen-oxidizing bacteria 21, which is an example of the microorganism, from the culture solution 18. By providing the circulation path 54 independently of the recovery path 56, it is possible to reduce the parameters that affect the average flow velocity of the culture solution 18, and thus, it is easy to control the average flow velocity of the culture solution 18. In addition, in a case where the sparger 28 is disposed in the recovery path 56, there is a concern that the raw material gas 26 that has been supplied is recovered before being supplied to the culture tank 11. By providing the circulation path 54 separately from the recovery path 56, it is possible to suppress a decrease in the utilization efficiency of such a raw material gas 26.

In addition, in the above-described embodiment, the microorganism as the culture target of the culture apparatus 10 is the hydrogen-oxidizing bacteria 21, and the raw material gas 26 contains hydrogen as a combustible gas and contains oxygen and carbon dioxide as other components. By having such a composition, the raw material gas 26 can culture the hydrogen-oxidizing bacteria 21.

In addition, in the above-described embodiment, each component of hydrogen, oxygen, and carbon dioxide of the raw material gas 26 is separately supplied. Therefore, it is easy to adjust the supply amount of the required components as the raw material gas 26. In addition, in a case where each component of the raw material gas 26 is separately supplied in this way, it is necessary to provide a plurality of spargers 28 (an example of a bubble generating device) for each component. In a case where a plurality of devices having a large energy consumption, such as a bubble generating device that generates microbubbles or nanobubbles, are provided, the energy consumption required for reducing the size of the bubbles is enormous due to the increase in the number. Therefore, in a case where a plurality of bubble generating devices are provided, the disclosed technology is very effective.

### (Compositional Ratio Control of Mixed Gas)

In addition, in the above-described embodiment, the culture apparatus 10 includes a compositional ratio control mechanism that measures a compositional ratio of a mixed gas 31 in a space 11A present above the liquid surface 18A of the culture solution 18 in the culture tank 11 and supplies an adjustment gas 33 to the space 11A according to the measured compositional ratio of the mixed gas 31 to maintain the compositional ratio of the mixed gas 31 in the space 11A at a target value outside a preset explosive range. By including such a compositional ratio control mechanism, the safety is further improved.

FIG. 8 shows an example of compositional ratio control. In the memory 17A of the processor 17, as a target value outside the explosive range used for the compositional ratio control, for example, a target value of the hydrogen concentration (TD(H₂)), a target value of the oxygen concentration (TD(O₂)), a water vapor concentration (not shown), a target temperature, and the like are stored.

As described above, hydrogen is a combustible gas, and the adjustment gas 33 is used to maintain the compositional ratio of the mixed gas 31 in the space 11A at a target value outside a preset explosive range. In the mixed gas 31, hydrogen, which is a combustible gas, is mixed with oxygen. In this case, an explosion occurs depending on the compositional ratio of the mixed gas 31. The compositional ratio of the mixed gas in a case where an explosion occurs is referred to as an explosive range. As described above, in the memory 17A of the processor 17, a target value of the hydrogen concentration (TD(H₂)), a target value of the oxygen concentration (TD(O₂)), a water vapor concentration, and the like are set as the target value outside the explosive range. The target value outside the explosive range is different depending on the component of the mixed gas 31. In a case where the mixed gas 31 is a mixed gas containing hydrogen and oxygen as in the present example, the target value outside the explosive range is generally such that the oxygen concentration in the mixed gas 31 is less than 5% or the hydrogen concentration is less than 4%.

Furthermore, the present applicant has verified through experiments that, in a case where the water vapor concentration is 7% or more, the compositional ratio of the mixed gas can be maintained outside the explosive range even in a case where the oxygen concentration is 5% or more. Therefore, in the present example, as an example, the target value outside the explosive range is set such that, in a case where the water vapor concentration is 7% or more and 90% or less, the oxygen concentration is set in a range of 5% or more and 9% or less.

The experiment was performed by the following method. As an explosion vessel, a stainless steel chamber having an inner diameter of 200 mm, a depth of 200 mm, and a volume of 6 L was used. The explosion vessel was filled with hydrogen, oxygen, carbon dioxide, and water vapor (the water vapor was filled by pouring water into the explosion vessel and performing vacuuming to a saturated water vapor pressure at a temperature in the container to vaporize the water). Hydrogen, oxygen, and carbon dioxide were filled from a gas cylinder through a gas introduction pipe provided in the explosion vessel). After rotating a stirring fan in the explosion vessel to uniformize the gas, an attempt was made to ignite the gas using a fine wire explosion device provided in the explosion vessel. The determination of ignition or non-ignition was performed based on the presence or absence of a pressure increase and the presence or absence of a negative pressure inside the explosion vessel due to the generation of water. The pressure was measured by a pressure sensor (PHS-B-10MP, manufactured by Kyowa Electronic Instruments Co., Ltd.) disposed on an upper valve of a gas discharge pipe provided in the explosion vessel.

Table 1 shows the experimental results of whether or not ignition occurs depending on the gas concentration of the mixed gas. Both Example 1 and Comparative Example 1 in Table 1 are examples in which the water vapor concentration is 0%, and the concentrations other than the oxygen concentration are substantially the same. In Example 1 of non-ignition, the oxygen concentration is 4%, which is less than 5%, and in Comparative Example 1 of ignition, the oxygen concentration is 9%, which is more than 5%. From Example 1 and Comparative Example 1, as is generally known, in a case where the water vapor concentration is 0%, the experimental result is that ignition does not occur in a case where the oxygen concentration is less than 5%.

In addition, Examples 2 and 3 and Comparative Example 2 are all examples in which the water vapor concentration is 7% or more, and only the oxygen concentration is significantly different. In Example 2 and Example 3, the oxygen concentration is 9%, and in Comparative Example 2, the oxygen concentration is 16%, which is more than 9%. From these experimental results, it can be seen that, in a case where the water vapor concentration is 7% or more, ignition does not occur even in a case where the oxygen concentration is 5% or more and 9% or less.

**[Table 1]**

| | Gas Concentration | | | | Temperature | Result |
|---|---|---|---|---|---|---|
| | H₂ | O₂ | CO₂ | H₂O | | |
| Example 1 | 72% | 4% | 24% | 0% | 31°C | Non-Ignition |
| Example 2 | 70% | 9% | 14% | 7% | 44°C | Non-Ignition |
| Example 3 | 66% | 9% | 13% | 12% | 52°C | Non-Ignition |
| Comparative Example 1 | 67% | 9% | 24% | 0% | 31°C | Ignition |
| Comparative Example 2 | 59% | 16% | 13% | 12% | 52°C | Ignition |

Although there is no experimental result in a case where the hydrogen concentration is less than 4% in Table 1, it is known that the mixed gas does not ignite even in a case where the water vapor concentration is 0% as long as the hydrogen concentration is less than 4% instead of setting the oxygen concentration to less than 5%. Therefore, as the target value outside the explosive range, the hydrogen concentration of the mixed gas 31 may be set to less than 4% instead of setting the oxygen concentration of the mixed gas 31 to less than 5%.

The adjustment gas 33 is, for example, an inert gas. More specifically, in the present example, carbon dioxide (CO₂) is used. By supplying the adjustment gas 33 to the space 11A, the oxygen concentration or the hydrogen concentration is reduced, and the compositional ratio of the mixed gas 31 is maintained at the target value outside the explosive range. As the adjustment gas 33, any gas can be used in addition to the inert gas. For example, as the adjustment gas 33, any gas may be used to set the compositional ratio of the mixed gas 31 to the target value outside the explosive range, such as using hydrogen, which is a combustible gas, to set the oxygen concentration of the mixed gas 31 to less than 5%. As described above, any gas can be used as the adjustment gas 33, but in a case where the mixed gas 31 is reused, it is preferable to use the component of the raw material gas 26 as the adjustment gas 33.

As shown in FIG. 8, the compositional ratio measuring unit 16 is disposed on the discharge path 36 for discharging the mixed gas 31 from the space 11A of the culture tank 11. The compositional ratio measuring unit 16 is composed of, for example, a gas analyzer (for example, manufactured by HORIBA, Ltd.: model number TCA-51d) that measures a hydrogen concentration (D(H₂)), a gas analyzer (for example, manufactured by HORIBA, Ltd.: model number VA-5113) that measures an oxygen concentration (D(O₂)), a gas analyzer (for example, manufactured by HORIBA, Ltd.: model number VA-5113) that measures a carbon dioxide concentration (D(CO₂)), and a flow meter (for example, manufactured by Emerson: model number CMFS007M) that measures a total flow rate (Qout(total)) of the mixed gas 31. As a result, the compositional ratio measuring unit 16 can measure the concentration of each component of the mixed gas 31 and the total flow rate (Qout(total)). The compositional ratio measuring unit 16 outputs each measured value to the processor 17 as a measurement signal.

The processor 17 determines the supply amount of the adjustment gas 33 such that the oxygen concentration of the mixed gas 31 is maintained at less than a preset upper limit value, for example, based on the measured value of the total flow rate and the oxygen concentration of the mixed gas 31. Then, the processor 17 controls the pump 22 to supply the adjustment gas 33 of the determined supply amount from the adjustment gas tank 14 to the space 11A. As a result, the supply amount (Qin(CO₂)) of carbon dioxide in the space 11A is increased, the oxygen concentration (D(O₂)) is relatively reduced, and the compositional ratio of the mixed gas 31 in the space 11A is maintained at the target value (TD(O₂)) outside the preset explosive range. Since the hydrogen concentration (D(H₂)) is also reduced by increasing the supply amount of the adjustment gas 33, the hydrogen concentration can also be maintained at the target value (TD(H₂)) outside the explosive range. The processor 17, the adjustment gas tank 14, and the pump 22 on the supply path 14A are examples of a "compositional ratio control mechanism" according to the disclosed technology.

In addition, since both the culture solution 18 and the space 11A are present in the culture tank 11, the temperature in the space 11A can also be maintained at substantially the same temperature by maintaining the temperature T of the culture solution 18 at the target temperature. As will be described later, the compositional ratio of the mixed gas 31 in the space 11A is maintained at the target value outside the preset explosive range. The target value also includes the water vapor concentration of the mixed gas 31. By controlling the temperature of the culture solution 18 to 40°C or higher, the temperature of the mixed gas 31 can be set to 40°C or higher. As a result, the water vapor concentration of the mixed gas 31 can also be adjusted to 7% or more.

In FIG. 8, the circulation path 54 is omitted or the sparger 28 is illustrated in the culture tank 11 in order to suppress the complication of the drawing, but the culture apparatus 10 of the first embodiment actually has the circulation path 54, and the sparger 28 is disposed in the circulation path 54.

### [Second Embodiment]

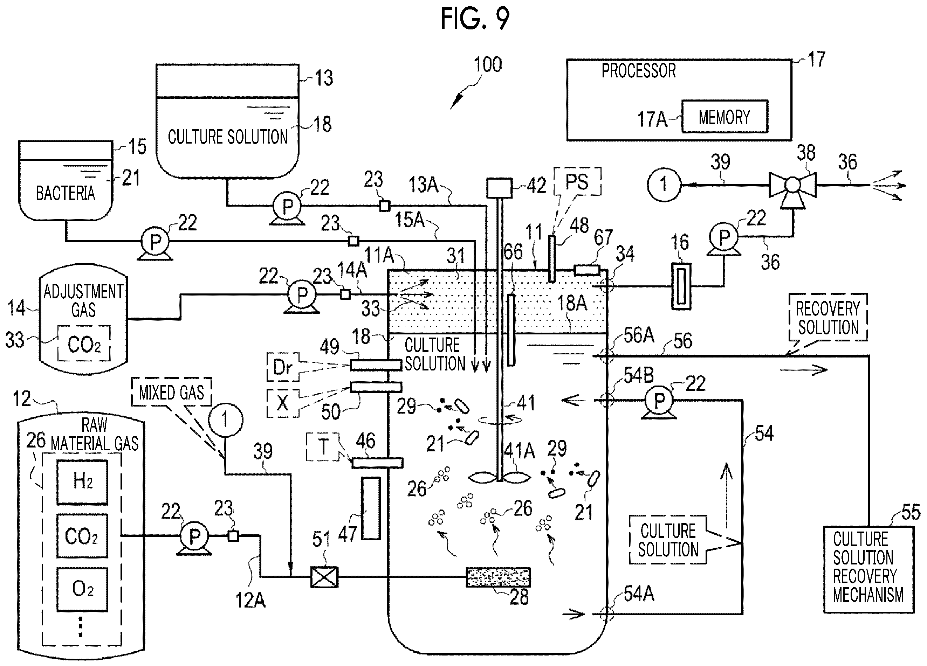

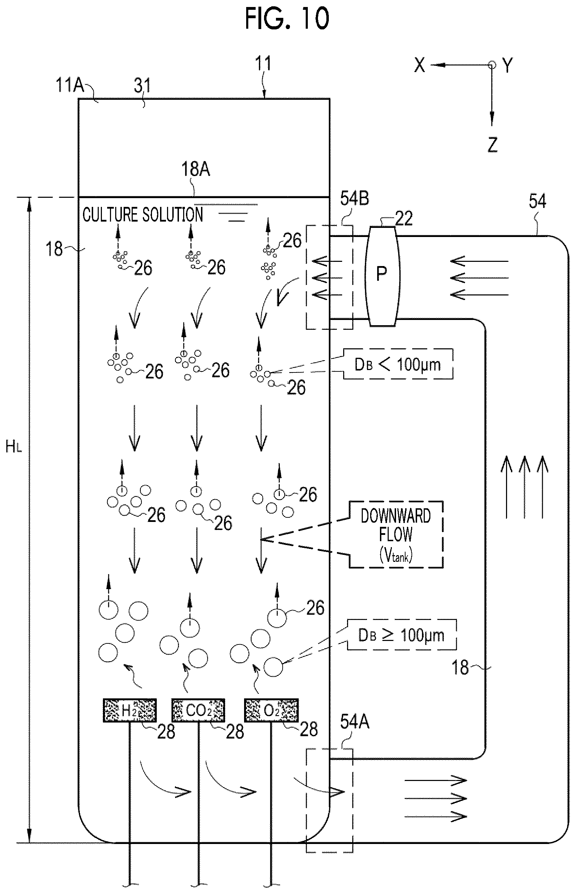

The differences between the culture apparatus 100 of the second embodiment shown in FIGS. 9 and 10 and the culture apparatus 10 of the first embodiment are that, first, the sparger 28 which is an example of a bubble generating device is disposed in the culture tank 11, and second, the direction in which the culture solution 18 circulates is reversed. Since the other configurations are the same as those of the first embodiment, the description thereof will be omitted, and the differences will be mainly described below. In FIG. 10, similarly to FIG. 4 of the first embodiment, for convenience, only the components necessary for describing the circulation of the culture solution 18 are illustrated, and the other configurations are omitted in order to suppress the complication of the drawing.

As shown in FIG. 10, the sparger 28 is disposed on the lower side of the culture tank 11 in the vertical direction (corresponding to the Z direction), that is, close to the bottom. As an example, the position of the sparger 28 is a position of half or less of the height H_{L} to the liquid surface 18A of the culture tank 11, and more preferably 1/4 or less. As described above, as the distance to the liquid surface 18A is longer, the residence time of the bubbles is longer, and thus it is preferable that the sparger 28 is disposed as close to the bottom as possible.

In addition, in the culture apparatus 100 of the second embodiment, the sparger 28 is a bubble generating device that generates bubbles having a diameter of 100 µm or more, and supplies the raw material gas 26 to the culture tank 11 as bubbles, which is the same as in the first embodiment.

In addition, although the sparger 28 is shown in a simplified manner in FIG. 9, as shown in FIG. 10, the sparger 28 is provided for each component of the raw material gas 26, and the supply amount can be controlled for each component such as hydrogen, carbon dioxide, and oxygen. This point is also the same as in the first embodiment, and the same effect as in the first embodiment can be obtained.

In the culture apparatus 100 of the second embodiment, the circulation direction of the culture solution 18 is opposite to that of the first embodiment. That is, the outlet port 54A of the circulation path 54 is provided in the vicinity of the bottom of the culture tank 11 and is disposed below the return port 54B in the vertical direction. In the culture apparatus 100 of the second embodiment, the flow of the culture solution 18 in the circulation path 54 is an upward flow flowing upward in the vertical direction.

In addition, the pump 22 is provided in the vicinity of the return port 54B of the circulation path 54. The pump 22 is a pump that generates a pressure for circulating the culture solution 18 through the circulation path 54, and is a pump that generates a downward flow from an upper side to a lower side in the vertical direction as a flow of the culture solution 18 in the culture tank 11.

That is, in the culture apparatus 100 of the second embodiment, unlike the first embodiment, the flow of the culture solution 18 in the circulation path 54 is an upward flow, and the flow of the culture solution 18 in the culture tank 11 is a downward flow. Therefore, in the culture tank 11, the culture solution 18 flows in a direction opposite to the rising direction of the raw material gas 26. In the culture tank 11, the downward flow of the culture solution 18 acts as a resistance to the bubbles that are about to rise, thereby reducing the rising velocity of the bubbles. As a result, since the residence time of the bubbles until reaching the liquid surface 18A is longer, the dissolution of the bubbles in the culture solution 18 progresses. In the culture apparatus 100, since the residence time of the bubble diameter D_{B} is longer than that in a case where there is no downward flow of the culture solution 18 in the culture tank 11, even when the bubble diameter D_{B} immediately after the sparger 28 generates the bubbles is 100 µm or more, the bubble diameter D_{B} is likely to be less than 100 µm.

As described above, in the culture apparatus 100 of the second embodiment, even when the supplied bubble diameter is 100 µm or more, the dissolution rate can be improved. That is, also in the second embodiment, as in the first embodiment, the dissolution rate of the raw material gas can be improved while suppressing the energy consumption required for reducing the size of the bubbles.

Further, in the culture apparatus 100 of the second embodiment, it is preferable that the processor 17 controls the flow velocity of the downward flow of the culture solution 18 flowing in the culture tank 11 as follows. That is, in a case where the average flow velocity of the downward flow of the culture solution 18 in the culture tank 11 is denoted by Vₜₐₙₖ, it is preferable to control Vₜₐₙₖ to satisfy a condition of Expression (7).$\frac{{H}_{L}}{{V}_{B} - {V}_{tank}} > 1 1 . 2 \left[S\right]$ (7)

Here, as in the first embodiment, H_{L} is the height of the liquid surface of the culture solution in the culture tank, and V_{B} is the rising velocity of the bubbles.

Expression (7) is a condition required to secure the minimum time required for practical use from the viewpoint of securing the dissolution rate of the bubbles as the residence time of the bubbles in the culture tank 11, as in Expression (4) of the first embodiment. The basis for the value of 11.2 seconds is the same as in the first embodiment. Vₜₐₙₖ is controlled such that the residence time is longer than 11.2 seconds.

In addition, in the culture apparatus 100 of the second embodiment, as shown in FIG. 10, it is preferable that the outlet port 54A of the circulation path 54 is disposed below the sparger 28, which is an example of the bubble generating device, in the vertical direction. By disposing the outlet port 54A below the sparger 28, it is possible to suppress the bubbles immediately after being generated from the sparger 28 from being drawn into the circulation path 54. Since the bubbles immediately after being generated from the sparger 28 have a large diameter, when the bubbles are drawn into the circulation path 54, bubbles having a large diameter flow into the culture tank 11 from the return port 54B above the outlet port 54A. Then, the residence time of the bubbles of the raw material gas 26 is shortened. In addition, since the amount of the raw material gas 26 that is not dissolved in the culture solution 18 and is released into the space 11A increases, the utilization efficiency of the raw material gas 26 also deteriorates. By disposing the outlet port 54A below the sparger 28, such a problem can be suppressed.

In addition, the circulation path 54 of the second embodiment is a path dedicated to circulation and provided independently of the recovery path 56, as in the first embodiment. Therefore, as in the first embodiment, an effect of easily controlling the flow velocity can be obtained. In addition, in the second embodiment, the compositional ratio control shown in FIG. 8 in the first embodiment may be performed.

Further, as shown in FIG. 6 of the first embodiment, in the second embodiment as well, the direction in which the circulation path 54 extends may be inclined with respect to the vertical direction such that the positions of the outlet port 54A and the return port 54B of the circulation path 54 are disposed at different positions in the width direction of the culture tank 11. In this manner, a flow in which the culture solution 18 revolves along the circumferential direction in the culture tank 11 can be generated, and the unevenness in the concentration of the raw material gas 26 in the culture tank 11 can be suppressed.

As described in the first embodiment, the circulation path 54 may have a shape of a curve (including a spiral) in addition to a linear shape, in whole or in part. By adopting such a shape, the circulation path 54 can be flexibly disposed according to the space around the culture tank 11. In addition, by adopting a helical shape, a longer circulation path 54 can be disposed in the same space. By increasing the length of the circulation path 54, the residence time of the bubbles can be increased.

In each of the above-described embodiments, the hydrogen-oxidizing bacteria 21 have been described as an example of the microorganism, but the disclosed technology can also be applied to microorganisms or cells other than the hydrogen-oxidizing bacteria 21. The microorganism may be, for example, an acetogenic bacterium such as Clostridium autoethanogenum, or a methanotrophic bacterium such as Methylococcus capsulatus. In a case of the acetogenic bacterium, the components of the raw material gas 26 are hydrogen (H₂), carbon monoxide (CO), and carbon dioxide (CO₂), which are combustible gases. In a case of the methanotrophic bacteria, the components of the raw material gas 26 are methane (CH₄), which is a combustible gas, and air. In addition, the cell is a morphological and/or functional unit constituting a living body, and the living body from which the cell is derived is not particularly limited, but may be an animal, a plant, or the like. The cells may be, for example, established cells and genetically modified cells, and may include artificial cells. Examples thereof include Chinese hamster ovary cells (CHO cells), Human Embryonic Kidney cells 293 (HEK293 cells), and Spodoptera frugiperda cells 9 (Sf9 cells).

In addition, the above-described embodiments disclose the technologies according to the following first supplementary note and second supplementary note.

### [First Supplementary Note 1]

A culture apparatus for culturing a microorganism or a cell that uses a raw material gas containing a combustible gas for proliferation or production of an organic substance, the culture apparatus comprising:
a culture tank that contains a culture solution for culturing the microorganism or the cell;
a circulation path that takes out the culture solution from an outlet port provided in the culture tank and returns the taken-out culture solution to the culture tank from a return port provided in the culture tank;
a pump that generates a pressure for circulating the culture solution through the circulation path to generate, as a flow of the culture solution in the circulation path, a downward flow from an upper side toward a lower side in a vertical direction; and
a bubble generating device that generates bubbles having a diameter of 100 µm or more to supply the raw material gas to the circulation path as bubbles,
wherein the outlet port is disposed above the return port in the vertical direction, and
the bubble generating device is disposed on an upstream side of a midpoint of a length from the outlet port to the return port.

### [First Supplementary Note 2]

The culture apparatus according to First Supplementary Note 1, wherein, in a case where a rising velocity of the bubbles is denoted by V_{B} and an average flow velocity of the downward flow of the culture solution in the circulation path is denoted by Vₚᵢₚₑ, Vₚᵢₚₑ satisfies a condition of the following expression, ${V}_{pipe} > {V}_{B} .$

### [First Supplementary Note 3]

The culture apparatus according to First Supplementary Note 2, in which, in a case where a height in the vertical direction from the return port to the bubble generating device in the circulation path is denoted by Hₚᵢₚₑ, a height from a bottom of the culture tank to a liquid surface of the culture solution is denoted by H_{L}, a height from the bottom of the culture tank to the return port is denoted by Hᵢₙ, and a rising velocity of the bubbles is denoted by V_{B}, Vₚᵢₚₑ satisfies a condition of the following expression, ${V}_{pipe} < \left(1+\frac{{H}_{pipe}}{{V}_{B} ⋅ 1 1.2 \left[S\right] - {H}_{L} + {H}_{in}}\right) {V}_{B} .$

### [First Supplementary Note 4]

The culture apparatus according to any one of First Supplementary Notes 1 to 3,
wherein the circulation path is a path dedicated to circulation and provided independently of a recovery path for recovering a part of the microorganism or the cell from the culture solution.

### [First Supplementary Note 5]

The culture apparatus according to any one of First Supplementary Notes 1 to 4,
wherein the return port is provided in a range of 1/4 or less of a height of the liquid surface from a bottom in the culture tank.

### [First Supplementary Note 6]

The culture apparatus according to any one of First Supplementary Notes 1 to 4,
wherein the microorganism is hydrogen-oxidizing bacteria, and the raw material gas contains hydrogen as the combustible gas and contains oxygen and carbon dioxide as other components.

### [First Supplementary Note 7]

The culture apparatus according to any one of First Supplementary Notes 1 to 6,
wherein the raw material gas contains hydrogen as the combustible gas and contains oxygen and carbon dioxide as other components, and
each component of the hydrogen, the oxygen, and the carbon dioxide is separately supplied.

### [First Supplementary Note 8]

The culture apparatus according to any one of First Supplementary Notes 1 to 7,
wherein the culture apparatus further includes a compositional ratio control mechanism that measures a compositional ratio of a mixed gas in a space present above a liquid surface of the culture solution in the culture tank and supplies an adjustment gas to the space according to the measured compositional ratio of the mixed gas to maintain the compositional ratio of the mixed gas in the space at a target value outside a preset explosive range.

### [Second Supplementary Note 1]

A culture apparatus for culturing a microorganism or a cell that uses a raw material gas containing a combustible gas for proliferation or production of an organic substance, the culture apparatus comprising:
a culture tank that contains a culture solution for culturing the microorganism or the cell;
a circulation path that takes out the culture solution from an outlet port provided in the culture tank and returns the taken-out culture solution to the culture tank from a return port provided in the culture tank;
a pump that generates a pressure for circulating the culture solution through the circulation path to generate, as a flow of the culture solution in the culture tank, a downward flow from an upper side toward a lower side in a vertical direction; and
a bubble generating device that generates bubbles having a diameter of 100 µm or more to supply the raw material gas to the culture tank as bubbles.

### [Second Supplementary Note 2]

The culture apparatus according to Second Supplementary Note 1, wherein, in a case where a height of a liquid surface of the culture solution in the culture tank is denoted by H_{L}, a rising velocity of the bubbles is denoted by V_{B}, and an average flow velocity of the downward flow of the culture solution in the culture tank is denoted by Vₜₐₙₖ, Vₜₐₙₖ satisfies a condition of the following expression, $\frac{{H}_{L}}{{V}_{B} - {V}_{tank}} > 1 1 . 2 \left[S\right] .$

### [Second Supplementary Note 3]

The culture apparatus according to Second Supplementary Note 1 or 2,
wherein the circulation path is a path dedicated to circulation and provided independently of a recovery path for recovering a part of the microorganism or the cell from the culture solution.

### [Second Supplementary Note 4]

The culture apparatus according to any one of Second Supplementary Notes 1 to 3,
wherein the outlet port is disposed below the bubble generating device.

### [Second Supplementary Note 5]

The culture apparatus according to any one of Second Supplementary Notes 1 to 4,
wherein the microorganism is hydrogen-oxidizing bacteria, and the raw material gas contains hydrogen as the combustible gas and contains oxygen and carbon dioxide as other components.

### [Second Supplementary Note 6]

The culture apparatus according to any one of Second Supplementary Notes 1 to 5,
wherein the raw material gas contains hydrogen as the combustible gas and contains oxygen and carbon dioxide as other components, and
the raw material gas is supplied such that each component of the hydrogen, the oxygen, and the carbon dioxide is separately supplied.

### [Second Supplementary Note 7]

The culture apparatus according to any one of Second Supplementary Notes 1 to 6,
wherein the culture apparatus further comprises a compositional ratio control mechanism that measures a compositional ratio of a mixed gas in a space present above a liquid surface of the culture solution in the culture tank and supplies an adjustment gas into the space according to the measured compositional ratio of the mixed gas to maintain the compositional ratio of the mixed gas in the space at a target value outside a preset explosive range.

The above-described processor includes, in addition to a general-purpose processor such as a CPU that executes software (program) to function as various processing units, a programmable logic device (PLD) such as a field programmable gate array (FPGA) that is a processor having a circuit configuration changeable after manufacture, a dedicated electric circuit such as an application specific integrated circuit (ASIC) that is a processor having a circuit configuration dedicatedly designed to execute specific processing, and the like.

Further, as the hardware structure of the various processors, more specifically, an electrical circuit (circuitry) in which circuit elements such as semiconductor elements are combined can be used.

The above-described contents and the above-shown contents are detailed descriptions of portions according to the disclosed technology, and are merely examples of the disclosed technology. For example, the above-described descriptions of the configurations, the functions, the operations, and the effects are descriptions of examples of the configurations, the functions, the operations, and the effects of the portions according to the disclosed technology. Therefore, it goes without saying that unnecessary parts may be deleted, new elements may be added, or replacements may be made with respect to the above-described contents and the above-shown contents within a range that does not deviate from the gist of the disclosed technology. In addition, in order to avoid complication and to facilitate understanding of the portions according to the disclosed technology, the above-described contents and the above-shown contents do not include descriptions of common technical knowledge and the like that do not require particular description for enabling the disclosed technology.

The disclosure of JP2024-030603 filed on February 29, 2024 is incorporated herein by reference in its entirety into the present specification. In addition, all of the documents, the patent applications, and the technical standards described in the present specification are incorporated herein by reference to the same extent as in a case in which each of the documents, the patent applications, and the technical standards are specifically and individually noted to be incorporated by reference.

## Claims

1. A culture apparatus for culturing a microorganism or a cell that uses a raw material gas containing a combustible gas for proliferation or production of an organic substance, the culture apparatus comprising:
a culture tank that contains a culture solution for culturing the microorganism or the cell;
a circulation path that takes out the culture solution from an outlet port provided in the culture tank and returns the taken-out culture solution to the culture tank from a return port provided in the culture tank;
a pump that generates a pressure for circulating the culture solution through the circulation path to generate, as a flow of the culture solution in the circulation path, a downward flow from an upper side toward a lower side in a vertical direction; and
a bubble generating device that generates bubbles having a diameter of 100 µm or more to supply the raw material gas to the circulation path as bubbles,
wherein the outlet port is disposed above the return port in the vertical direction, and
the bubble generating device is disposed on an upstream side of a midpoint of a length from the outlet port to the return port.

2. The culture apparatus according to claim 1,wherein, in a case where an average flow velocity of the downward flow of the culture solution in the circulation path is denoted by Vₚᵢₚₑ and a rising velocity of the bubbles is denoted by V_{B}, Vₚᵢₚₑ satisfies a condition of the following expression, ${V}_{pipe} > {V}_{B} .$

3. The culture apparatus according to claim 2,wherein, in a case where a height in the vertical direction from the return port to the bubble generating device in the circulation path is denoted by Hₚᵢₚₑ, a height from a bottom of the culture tank to a liquid surface of the culture solution is denoted by H_{L}, and a height from the bottom of the culture tank to the return port is denoted by Hᵢₙ, Vₚᵢₚₑ satisfies a condition of the following expression, ${V}_{pipe} < \left(1+\frac{{H}_{pipe}}{{V}_{B} ⋅ 1 1.2 \left[S\right] - {H}_{L} + {H}_{in}}\right) {V}_{B} .$

4. The culture apparatus according to claim 1,
wherein the circulation path is a path dedicated to circulation and provided independently of a recovery path for recovering part of the microorganism or the cell from the culture solution.

5. The culture apparatus according to claim 1,
wherein the return port is provided in a range of 1/4 or less of a height of a liquid surface from a bottom in the culture tank.

6. The culture apparatus according to claim 1,
wherein the microorganism is hydrogen-oxidizing bacteria, and the raw material gas contains hydrogen as the combustible gas and contains oxygen and carbon dioxide as other components.

7. The culture apparatus according to claim 1,
wherein the raw material gas contains hydrogen as the combustible gas and contains oxygen and carbon dioxide as other components, and
each component of the hydrogen, the oxygen, and the carbon dioxide is separately supplied.

8. The culture apparatus according to claim 1, further comprising a compositional ratio control mechanism that measures a compositional ratio of a mixed gas in a space present above a liquid surface of the culture solution in the culture tank and supplies an adjustment gas into the space according to the measured compositional ratio of the mixed gas to maintain the compositional ratio of the mixed gas in the space at a target value outside a preset explosive range.

9. A culture apparatus for culturing a microorganism or a cell that uses a raw material gas containing a combustible gas for proliferation or production of an organic substance, the culture apparatus comprising:
a culture tank that contains a culture solution for culturing the microorganism or the cell;
a circulation path that takes out the culture solution from an outlet port provided in the culture tank and returns the taken-out culture solution to the culture tank from a return port provided in the culture tank;
a pump that generates a pressure for circulating the culture solution through the circulation path to generate, as a flow of the culture solution in the culture tank, a downward flow from an upper side toward a lower side in a vertical direction; and
a bubble generating device that generates bubbles having a diameter of 100 µm or more to supply the raw material gas to the culture tank as bubbles.

10. The culture apparatus according to claim 9,wherein, in a case where a height of a liquid surface of the culture solution in the culture tank is denoted by H_{L}, an average flow velocity of the downward flow of the culture solution in the culture tank is denoted by Vₜₐₙₖ, and a rising velocity of the bubbles is denoted by V_{B}, Vₜₐₙₖ satisfies a condition of the following expression, $\frac{{H}_{L}}{{V}_{B} - {V}_{tank}} > 1 1 . 2 \left[S\right] .$

11. The culture apparatus according to claim 9,
wherein the circulation path is a path dedicated to circulation and provided independently of a recovery path for recovering a part of the microorganism or the cell from the culture solution.

12. The culture apparatus according to claim 1,
wherein the outlet port is disposed below the bubble generating device.

13. The culture apparatus according to claim 9,
wherein the microorganism is hydrogen-oxidizing bacteria, and the raw material gas contains hydrogen as the combustible gas and contains oxygen and carbon dioxide as other components.

14. The culture apparatus according to claim 1,
wherein the raw material gas contains hydrogen as the combustible gas and contains oxygen and carbon dioxide as other components, and
the raw material gas is supplied such that each component of the hydrogen, the oxygen, and the carbon dioxide is separately supplied.

15. The culture apparatus according to claim 9, further comprising a compositional ratio control mechanism that measures a compositional ratio of a mixed gas in a space present above a liquid surface of the culture solution in the culture tank and supplies an adjustment gas into the space according to the measured compositional ratio of the mixed gas to maintain the compositional ratio of the mixed gas in the space at a target value outside a preset explosive range.
